(19) Europäisches Patentamt
European Patent Office
Office européen des brevets

(11) EP 2 061 864 B1

(12) **EUROPÄISCHE PATENTSCHRIFT**

(45) Veröffentlichungstag und Bekanntmachung des Hinweises auf die Patenterteilung:
**11.05.2011 Patentblatt 2011/19**

(21) Anmeldenummer: **07821179.4**

(22) Anmeldetag: **11.10.2007**

(51) Int Cl.:
*C11D 3/02* (2006.01) *C11D 3/12* (2006.01)
*C11D 3/20* (2006.01) *C11D 3/22* (2006.01)
*C11D 3/37* (2006.01) *C11D 3/50* (2006.01)
*C11D 17/00* (2006.01)

(86) Internationale Anmeldenummer:
**PCT/EP2007/060812**

(87) Internationale Veröffentlichungsnummer:
**WO 2008/058819 (22.05.2008 Gazette 2008/21)**

(54) **FESTE, TEXTIL- UND/ODER HAUTPFLEGENDE ZUSAMMENSETZUNG**

SOLID TEXTILE CARE AND/OR SKINCARE COMPOSITION

COMPOSITION SOLIDE, TEXTILE ET/OU DE SOIN DE LA PEAU

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **16.11.2006 DE 102006054436**

(43) Veröffentlichungstag der Anmeldung:
**27.05.2009 Patentblatt 2009/22**

(73) Patentinhaber: **Henkel AG & Co. KGaA**
**40589 Düsseldorf (DE)**

(72) Erfinder:
- **MAYER, Konstanze**
  **40589 Düsseldorf (DE)**
- **SCHEFFLER, Karl-Heinz**
  **40589 Düsseldorf (DE)**
- **ARTIGA GONZALEZ, Rene-Andres**
  **40589 Düsseldorf (DE)**
- **STURM, Mario**
  **51371 Leverkusen (DE)**

(56) Entgegenhaltungen:
**EP-A- 0 215 637** **EP-A- 0 483 411**
**EP-A- 1 561 802** **WO-A-00/17298**
**WO-A-03/055966** **WO-A-2006/053598**
**GB-A9- 2 331 305** **US-A- 4 539 135**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft eine feste, textil- und/oder hautpflegende Zusammensetzung sowie deren Verwendung und Herstellung. Desweiteren betrifft die Erfindung ein Wasch- oder Reinigungsmittel, das die feste, textil- und/oder hautpflegende Zusammensetzung enthält.

Durch wiederholtes Waschen werden Textilien oft hart und verlieren ihre Weichheit. Um Textilien ihre Weichheit/ Flexibilität wiederzugeben, um ihnen einen angenehmen Duft zu verleihen und/oder um ihre antistatischen Eigenschaften zu verbessern, werden die Textilien nach dem eigentlichen Wasch- und Reinigungsprozess in einem anschließenden Spülprozess mit einem Weichspüler behandelt.

Die meisten, im Markt befindlichen Weichspüler sind wässrige Formulierungen, die als Hauptwirkungsbestandteil eine kationische Textil-weichmachende Verbindung, die eine oder zwei langkettige Alkylgruppen in einem Molekül aufweist, enthalten. Weit verbreitete kationische Textil-weichmachende Verbindungen umfassen beispielsweise Methyl-N-(2-hydroxyethyl)-N,N-di(talg-acyloxyethyl)ammonium-Verbindungen, Methyl-N-(2-hydroxyethyl)-N,N-di(talgacyloxyethyl)ammoniumverbindungen oder N,N-Dimethyl-N,N-di(talgacyloxyethyl)ammonium-Verbindungen.

Diese herkömmlichen Weichspülerformulierungen können wegen der kationischen, Textil-weichmachenden Verbindungen nicht gleichzeitig mit dem Wasch- oder Reinigungsmittel im eigentlichen Wasch- oder Reinigungsprozess verwendet werden, da die kationischen Textil-weichmachenden Verbindungen mit den anionischen Tensiden der Wasch- oder Reinigungsmittel unerwünscht wechselwirken. Deshalb ist ein zusätzlicher Spülvorgang notwendig, der aber zeit- und energieintensiv ist.

Ein weiterer Nachteil ist, dass herkömmliche Weichspüler nicht die Ablagerung von Kalkrückständen während des Spülvorgangs auf der Wäsche verhindern. Zusätzlich hinterlassen die herkömmlichen Weichspüler oft unschöne Ablagerung in der Einspülkammer der Waschmaschine.

Es ist deshalb eine Aufgabe der vorliegenden Erfindung, eine textil- und/oder hautpflegende Zusammensetzung bereitzustellen, die im Hauptwaschgang zusammen mit Wasch- oder Reinigungsmitteln eingesetzt werden kann.

Diese Aufgabe wird gelöst durch eine feste, textil- und/oder hautpflegende Zusammensetzung, umfassend einen wasserlöslichen Partikel und einen wasserunlöslichen Partikel, der einen wasserunlöslichen Träger und 0,1 bis 20 Gew. % einer textil- und/oder hautpflegenden Verbindung enthält, wobei der wasserlösliche Partikel ein kohlenhydrat umfasst, die textil- und/oder hautpflegende verbindung ein Parfüm enthält und der wasserunlösliche Träger ein textil-weichmachender Ton ist.

Eine solche textil- und/oder hautpflegende Zusammensetzung kann im Hauptwaschgang eines automatischen Wasch- oder Reinigungsverfahrens eingesetzt werden. Die textil- und/oder hautpflegende Zusammensetzung kann beispielsweise zusammen mit dem Wasch- oder Reinigungsmittel in die Trommel oder die Einspülkammer einer Waschmaschine gegeben werden. Dies hat den Vorteil, dass kein zusätzlicher Spülgang notwendig ist und keine unschönen Ablagerungen in der Einspülkammer auftreten.

Weiterhin ist vorteilhaft, dass die textil- und/oder hautpflegende Verbindung bereits direkt zu Beginn des Waschverfahrens zur Wäsche transportiert werden und so ihr volles Potential entfalten kann.

Da Konsumenten, insbesondere bei konzentrierten Produkten, dazu neigen überzudosieren und eine Überdosierung nicht nur zu unschönen Ablagerungen auf den Textilien, sondern auch zu Frustrationen beim Anwender bezüglich der Ergiebigkeit eines Produktes führen kann, enthält die textil- und/oder hautpflegende Zusammensetzung wasserlösliche Partikel als Füllstoffe.

Es ist bevorzugt, dass der wasserlösliche Partikel eine Verbindung ausgewählt aus der Gruppe bestehend aus anorganischen Alkalimetallsalzen, organischen Alkalimetallsalzen, anorganischen Erdalkalimetallsalzen, organischen Erdalkalimetallsalzen, organischen Säuren, Kohlenhydraten, Silikaten und Mischungen daraus enthält.

Diese Materialien sind nicht nur preiswert, sondern lösen sich sehr gut in Wasser. Außerdem sind diese Materialien geruchsneutral.

Der wasserlösliche Partikel umfasst ein Kohlenhydrat und ist ausgewählt aus der Gruppe bestehend aus Dextrose, Fructose, Galactose, Isoglucose, Glucose, Saccharose, Raffinose und Mischungen daraus.

Bei Verwendung eines wasserlöslichen Partikels, der aus Kohlenhydraten bzw. zumindest überwiegend aus Kohlenhydraten besteht, wird das Problem der Korrosion in der Waschmaschine vermieden, welches insbesondere bei Verwendung von anorganischen Salzen als wasserlöslicher Partikel auftreten kann.

Es ist vorteilhaft, dass die feste, textil- und/oder hautpflegende Zusammensetzung 10 bis 90 Gew.-%, bevorzugt 40 bis 60 Gew.-% und ganz besonders bevorzugt 45 bis 55 Gew.-%, an wasserlöslichen Partikeln enthält.

Der wasserunlösliche Träger ist ein Textil-weichmachender Ton, insbesondere ein Bentonit.

Textil-weichmachende Tone eignen sich besonders gut als Träger für andere Verbindungen, da Tone diese gut absorbieren bzw. adsorbieren können und damit behandelten Textilien einen Weichgriff vermitteln. Sie weisen auch einen Wasser-enthärtenden Effekt aufweisen, so dass bei ihrer Verwendung zusätzlich Kalkablagerungen auf der Wäsche verhindert werden.

Es ist insbesondere bevorzugt, dass eine weitere textil- und/oder hautpflegende Verbindung enthalten ist, die

ausgewählt ist aus der Gruppe, bestehend aus Textil-weichmachenden Verbindungen, Fluoreszenzmitteln, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Germiziden, Fungiziden, Antioxidantien, Antistatika, Bügelhilfsmitteln, UV-Absorbern, Phobiermitteln, Imprägniermitteln, hautpflegenden Verbindungen, und Mischungen daraus.

Den mit der erfindungsgemäßen textil- und/oder hautpflegenden Zusammensetzung behandelten Textilien wird durch die Zugabe einer oder mehr dieser textil- und/oder hautpflegenden Verbindungen ein vorteilhafter Effekt vermittelt bzw. werden für die Textilien schädliche oder negative Effekte, die beim Reinigen und/oder Konditionieren und/oder Tragen auftreten können, wie beispielsweise Verblassen, Vergrauung, usw. vermindert.

Es ist ganz besonders bevorzugt, dass der wasserunlösliche Partikel als Textil-weichmachende Verbindung ein Textil-weichmachendes Polymer ausgewählt aus der Gruppe, bestehend aus Polysiloxanen, kationischen Polymeren und Mischungen daraus enthält.

Durch die Zugabe eines Textil-weichmachenden Polymers, insbesondere eines Polysiloxans, eines kationischen Polymers oder einer Mischung daraus, kann der textil- und/oder hautpflegenden Zusammensetzung ein besonders guter Textil-weichmachender Effekt verliehen werden.

Der wasserunlösliche Partikel enthält als textil- und/oder hautpflegende Verbindung ein Parfüm. Dabei ist es insbesondere bevorzugt, dass die Menge an Parfüm 0,1 bis 20 Gew.-%, bevorzugt 1 bis 10 Gew.-% und insbesondere bevorzugt 2 bis 7 Gew.-% beträgt.

Textil- und/oder hautpflegende Zusammensetzungen sollen zumeist der Wäsche auch einen angenehmen und langanhaltenden Duft verleihen und enthalten aus diesem Grund bevorzugt ein Parfüm. Der Parfümeindruck der Wäsche kann, dabei durch, in der textil- und/oder hautpflegenden Zusammensetzung gegebenenfalls vorhandene, Polysiloxane und/oder kationischen Polymere verstärkt werden. Ein weiterer Vorteil der erfindungsgemäßen textil- und/oder hautpflegenden Zusammensetzung, die im Hauptwaschgang eingesetzt wird, ist, dass das Parfüm bereits direkt zu Beginn des Wasch- und Reinigungsverfahrens zur Wäsche transportiert wird und so sein volles Potential entfalten kann.

Bei herkömmlichen flüssigen Weichspülerzusammensetzungen mit quaternären Ammoniumverbindungen als Textil-weichmachende Verbindung tritt zudem bei höheren Parfümkonzentrationen (> 0,4 Gew.-% Parfüm bei regulären Weichspülerzusammensetzungen und ≥ 1 Gew.-% bei konzentrierten Weichspülerzusammensetzungen) auch ein Problem mit der Stabilität der Zusammensetzung auf. Bei den erfindungsgemäßen textil- und/oder hautpflegenden Zusammensetzungen können problemlos größere Mengen (≥ 1 Gew.-%) an Parfüm eingearbeitet werden.

Es ist weiterhin bevorzugt, dass der wasserunlösliche Partikel zusätzlich Inhaltsstoffe ausgewählt aus der Gruppe, bestehend aus Farbstoffen, Füllstoffen, Perlglanzmitteln und Mischungen daraus enthält.

Es ist insbesondere bevorzugt, dass der wasserlösliche Partikel und/oder der wasserunlösliche Partikel jeweils eine Partikelgröße im Bereich von 0,6 bis 30 mm, insbesondere 0,8 bis 20 mm und besonders bevorzugt 1 bis 10 mm, aufweisen.

Textil- und/oder hautpflegende Zusammensetzungen mit Partikelgrößen in diesen Bereichen lassen sich besonders gut und gezielt dosieren.

Die Erfindung betrifft auch die Verwendung einer erfindungsgemäßen festen, textil- und/oder hautpflegenden Zusammensetzung zum Konditionieren von textilen Flächengebilden.

Außerdem betrifft die Erfindung ein Verfahren zur Herstellung einer festen, textil- und/oder hautpflegenden Zusammensetzung, gemäss Anspruch 1 bei dem ein wasserlöslicher Partikel und ein wasserunlöslicher Partikel, der einen wasserunlöslichen Träger und eine textil- und/oder hautpflegenden Verbindung enthält, gemischt werden.

Ferner betrifft die Erfindung ein Wasch- oder Reinigungsmittel, umfassend eine erfindungsgemäße feste, textil- und/oder hautpflegende Zusammensetzung.

Durch das Einbringen der erfindungsgemäßen textil- und/oder hautpflegenden Zusammensetzung in ein Wasch- oder Reinigungsmittel können auf einfache und schnelle Weise, Wasch- oder Reinigungsmittel mit unterschiedlichen textil- und/oder hautpflegenden Effekten erhalten werden, da nur die Zusammensetzung der textil- und/oder hautpflegenden Zusammensetzung geändert werden muss. Weiterhin müssen beispielsweise bei Zugabe eines Parfüms zu der textil- und/oder hautpflegenden Zusammensetzung nicht das Wasch- oder Reinigungsmittel und die textil- und/oder hautpflegende Zusammensetzung parfümiert werden, sondern nur noch eines der beiden Mittel, vorzugsweise die textil- und/oder hautpflegende Zusammensetzung. Dies führt nicht nur zu geringeren Kosten, einem Wegfall der Überlagerung des Dufteindrucks bei Verwendung von zwei unterschiedlich parfümierten Produkten, sondern ist auch für Verbraucher mit empfindlicher Haut und/oder Allergien vorteilhaft.

Die Erfindung betrifft auch ein Verfahren zur Herstellung eines Wasch- oder Reinigungsmittels, umfassend eine erfindungsgemäße feste, textil- und/oder hautpflegende Zusammensetzung, bei dem ein festes Wasch- oder Reinigungsmittel mit einer erfindungsgemäßen festen, textil- und/oder hautpflegenden Zusammensetzung gemischt wird.

Gegenstand der Erfindung ist auch die Verwendung eines Wasch- oder Reinigungsmittels, umfassend eine erfindungsgemäße feste, textil- und/oder hautpflegende Zusammensetzung zum Reinigen und Konditionieren von textilen Flächengebilden.

Im Folgenden soll die Erfindung unter anderem anhand von Beispielen eingehender beschrieben werden.

Die feste, textil- und/oder hautpflegende Zusammensetzung enthält als essentielle Bestandteile einen wasserlöslichen Partikel und einen wasserunlöslichen Partikel. Der wasserunlösliche Partikel umfasst einen wasserunlöslichen Träger und eine textil- und/oder hautpflegende Verbindung.

"Wasserunlöslicher Partikel" bedeutet in diesem Zusammenhang, dass der Partikel nicht vollständig wasserlöslich ist und einen gewissen Anteil, vorzugsweise mindestens 10 Gew.-%, an wasserunlöslichen Verbindungen aufweist.

Der wasserunlösliche Träger des wasserunlöslichen Partikels ist ein Textil-weichmachender Ton, wie beispielsweise einen Smectit-Ton. Bevorzugte Smectit-Tone sind Beidellit-Tone, Hectorit-Tone, Laponit-Tone, Montmorillonit-Tone, Nontronit-Tone, Saponit-Tone, Sauconit-Tone und Mischungen daraus. Montmorillonit-Tone sind die bevorzugten weichmachenden Tone. Bentonite enthalten hauptsächlich Montmorillonite und können als bevorzugte Quelle für den Textil-weichmachenden Ton dienen.

Geeignete Bentonite werden beispielsweise unter den Bezeichnungen Laundrosil® von der Firma Süd-Chemie oder unter der Bezeichnung Detercal von der Firma Laviosa vertrieben.

Besonders bevorzugt ist der wasserunlösliche Träger ein granulierter Bentonit.

Die Menge an wasserunlöslichem Träger in dem wasserunlöslichen Partikel beträgt zwischen 10 und 90 Gew.-% und beträgt bevorzugt 40 bis 50 Gew.-%.

Neben dem wasserunlöslichen träger und dem Parfümkann der wasserunlösliche Partikel eine weitere textil- und/oder hautpflegende Verbindung enthalten Diese ist bevorzugt ausgewählt aus der Gruppe bestehend aus Textil-weichmachenden Verbindungen, Fluoreszenzmitteln, Antiredepositionsmittein, optischen Aufhellern, Vergrauungsinhibitoren, Einlaufverhinderern, Knitterschutzmitteln, Farbübertragungsinhibitoren, antimikrobiellen Wirkstoffen, Germiziden, Fungiziden, Antioxidantien, Antistatika, Bügelhilfsmitteln, UV-Absorbern, Phobiermitteln, Imprägniermitteln, hautpflegenden Verbindungen und Mischungen daraus.

Dabei ist es bevorzugt, dass die textil- und/oder hautpflegende Zusammensetzung als textil-weichmachende Verbindung ein Textil-weichmachendes Polymer, insbesondere ein Polysiloxan und/oder ein kationisches Polymer, enthält.

Ein bevorzugt einsetzbares Polysiloxan weist zumindest folgende Struktureinheit auf

a) 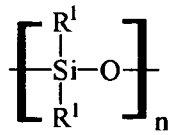

mit

$R^1$= unabhängig von einander $C_1$-$C_{30}$-Alkyl, vorzugsweise $C_1$-$C_4$-Alkyl, insbesondere Methyl oder Ethyl,

n = 1 bis 5000, vorzugsweise 10 bis 2500, insbesondere 100 bis 1500.

Es kann bevorzugt sein, dass das Polysiloxan zusätzlich auch folgende Struktureinheit aufweist:

b) 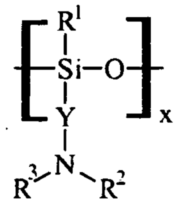

mit

R'= $C_1$-$C_{30}$-Alkyl, vorzugsweise $C_1$-$C_4$-Alkyl, insbesondere Methyl oder Ethyl,

Y = ggf. substituiertes, lineares oder verzweigtes $C_1$-$C_{20}$-Alkylen, vorzugsweise $-(CH_2)_m-$ mit m= 1 bis 16, vorzugsweise 1 bis 8, insbesondere 2 bis 4, im speziellen 3,

$R^2$, $R^3$ = unabhängig voneinander H oder gegebenenfalls substituiertes, lineares oder verzweigtes $C_1$-$C_{30}$-Alkyl, vorzugsweise mit Aminogruppen substituiertes $C_1$-$C_{30}$-Alkyl, besonders bevorzugt $-(CH_2)_b-NH_2$ mit b = 1 bis 10, äußerst bevorzugt b = 2,

x = 1 bis 5000, vorzugsweise 10 bis 2500, insbesondere 100 bis 1500.

Weist das Polysiloxan nur die Struktureinheit a) mit $R^1$ = Methyl auf, handelt es sich um ein Polydimethylsiloxan. Polydimethylpolysiloxane sind als effiziente Textil-pflegende Verbindungen bekannt.

Geeignete Polydimethysiloxane umfassen DC-200 (ex Dow Corning), Baysilone® M 50, Baysilone® M 100,

Baysilone® M 350, Baysilone® M 500, Baysilone® M 1000, Baysilone® M 1500, Baysilone® M 2000 oder Baysilone® M 5000 (alle ex GE Bayer Silicones).

Es kann allerdings auch bevorzugt sein, dass das Polysiloxan die Struktureinheiten a) und b) enthält. Ein besonders bevorzugtes Polysiloxan weist die folgende Struktur auf:

$$(CH_3)_3Si\text{-}[O\text{-}Si(CH_3)_2]_n\text{-}[O\text{-}Si(CH_3)\{(CH_2)_3\text{-}NH\text{-}(CH_2)_2\text{-}NH_2\}]_x\text{-}OSi(CH_3)_3$$

wobei die Summe n + x eine Zahl zwischen 2 und 10.000 ist.

Geeignete Polysiloxane mit den Struktureinheiten a) und b) sind beispielsweise kommerziell unter den Markennamen DC2-8663, DC2-8035, DC2-8203, DC05-7022 oder DC2-8566 (alle ex Dow Corning) erhältlich. Erfindungsgemäß ebenfalls geeignet sind beispielsweise die im Handel erhältlichen Produkte Dow Corning® 7224, Dow Corning® 929 Cationic Emulsion oder Formasil 410 (GE Silicones).

Geeignete kationische Polymere umfassen insbesondere solche, die in "CTFA International Cosmetic Ingredient Dictionary", Fourth Edition, J. M. Nikitakis, et al, Editors, veröffentlicht durch die Cosmetic, Toiletry, and Fragrance Association, 1991 beschrieben sind und unter der Sammelbezeichnung "Polyquaternium" zusammengefasst sind. Im Folgenden sind einige geeignete Polyquaternium-Verbindungen genauer aufgeführt.

POLYQUATERNIUM-1 (CAS-Nummer: 68518-54-7)
Definition: $\{(HOCH_2CH_2)_3N+\text{-}CH_2CH{=}CHCH_2\text{-}[N^+(CH_3)_2\text{-}CH_2CH{=}CHCH_2]_x\text{-}N^+(CH_2CH_2OH)_3\}[Cl^-]_{x+2}$

POLYQUATERNIUM-2 (CAS-Nummer: 63451-27-4)
Definition: $[\text{-}N(CH_3)_2\text{-}CH_2CH_2CH_2\text{-}NH\text{-}C(O)\text{-}NH\text{-}CH_2CH_2CH_2\text{-}N(CH_3)_2\text{-}CH_2CH_2OCH_2CH_2\text{-}]^{2+}\ (Cl^-)_2$
Beispielsweise erhältlich als Mirapol® A-15 (ex Rhodia)

POLYQUATERNIUM-3
Definition: Copolymer von Acrylamid und Trimethylammoniumethylmethacrylatmethosulfat

POLYQUATERNIUM-4 (CAS-Nummer: 92183-41-0)
Definition: Copolymer von Hydroxyethylcellulose und Diallyldimethylammoniumchlorid
Beispielsweise erhältlich als Celquat® H 100 oder Celquat® L200 (ex National Starch)

POLYQUATERNIUM-5 (CAS-Nummer: 26006-22-4)
Definition: Copolymer von Acrylamid und β-Methacrylyloxyethyltrimethylammoniummethosulfat.
Beispielsweise erhältlich als Nalco 7113 (ex Nalco) oder Reten® 210, Reten® 220, Reten® 230, Reten® 240, Reten® 1104, Reten® 1105 oder Reten® 1106 (alle ex Hercules)

POLYQUATERNIUM-6 (CAS-Nummer: 26062-79-3)
Definition: Polymer von Dimethyldiallylammoniumchlorid
Beispielsweise erhältlich als Merquat® 100 (ex Ondeo-Nalco)

POLYQUATERNIUM-7 (CAS-Nummer: 26590-05-6)
Definition: Polymeres quaternäres Ammoniumsalz bestehend aus Acrylamid- und Dimethyldiallylammoniumchlorid-Monomeren.
Beispielsweise erhältlich als Merquat® 550 oder Merquat® S (ex Ondeo-Nalco)

POLYQUATERNIUM-8
Definition: Polymeres quaternäres Ammoniumsalz von Methyl- und Stearyldimethylaminoethylmethacrylat, welches mit Dimethylsulfat quaternierte wurde.

POLYQUATERNIUM-9
Definition: Polymeres quaternäres Ammoniumsalz von Polydimethylaminoethylmethacrylat, welches mit Methylbromid quaternierte wurde.

POLYQUATERNIUM-10 (CAS-Numnern: 53568-66-4; 55353-19-0; 54351- 50-7; 81859-24-7; 68610-92-4; 81859-24-7)
Definition: Polymeres quaternäres Ammoniumsalz von Hydroxyethylcellulose, die mit einem Trimethylammonium-substitutierten Epoxid umgesetzt wurde.
Beispielsweise erhältlich als Celquat® SC-240 (ex National Starch), UCARE® Polymer JR-125, UCARE® Polymer

JR-400, UCARE® Polymer JR-30M, UCARE® Polymer LR 400, UCARE® Polymer LR 30M, Ucare® Polymer SR-10 (alle ex Amerchol)

POLYQUATERNIUM-11 (CAS-Nummer: 53633-54-8)
Definition: Quaternäres Ammoniumpolymer, welches durch Umsetzung von Diethylsulfat mit dem Copolymer von Vinylpyrrolidon und Dimethylaminoethylmethacrylat gebildet wird. Beispielsweise erhältlich als Luviquat®PQ 11 PN (ex BASF), Gafquat® 734, Gafquat® 755 oder Gafquat® 755N (ex GAF)

POLYQUATERNIUM-12 (CAS-Nummer: 68877-50-9)
Definition: Quaternäres Ammoniumpolymersalz, welches durch Umsetzung des Ethylmethacrylat/- Abietylmethacrylat/Diethylaminoethylmethacrylat-Copolymers mit Dimethylsulfat erhältlich ist

POLYQUATERNIUM-13 (CAS Nummer: 68877-47-4)
Definition: Polymeres quaternäres Ammoniumsalz, welches durch Umsetzung des Ethylmethacrylat/Oleylmethacrylat/Diethylaminoethylmethacrylat-Copolymers mit Dimethylsulfat erhältlich ist

POLYQUATERNIUM-14 (CAS-Nummer: 27103-90-8)
Definition: Polymeres quaternäres Ammoniumsalz der Formel $-\{-CH_2-C-(CH_3)-[C(O)O-CH_2CH_2-N(CH_3)_3^-]\}_x^+ [CH_3SO_4]^-_x$

POLYQUATERNIUM-15 (CAS-Nummer: 35429-19-7)
Definition: Copolymer von Acrylamid und β-Methacrylyloxyethyltrimethylammoniumchlorid

POLYQUATERNIUM-16 (CAS-Nummer: 95144-24-4)
Definition: Polymeres quaternäres Ammoniumsalz, gebildet aus Methylvinylimidazoliumchlorid und Vinylpyrrolidon Beispielsweise erhältlich als Luviquat® FC 370, Luviquat® Style, Luviquat® FC 550 oder Luviquat® Excellence (alle ex BASF)

POLYQUATERNIUM-17 (CAS-Nummer: 90624-75-2)
Definition: Polymeres quaternäres Ammoniumsalz, welches durch Umsetzung von Adipinsäure und Dimethylaminopropylamin mit Dichlorethylether erhältlich ist.
Beispielsweise erhältlich als Mirapol® AD-1 (ex Rhodia)

POLYQUATERNIUM-18
Definition: Polymeres quaternäres Ammoniumsalz, welches durch Umsetzung von Azelainsäure und Dimethylaminopropylamin mit Dichlorethylether erhältlich ist.
Beispielsweise erhältlich als Mirapol® AZ-1 (ex Rhodia)

POLYQUATERNIUM-19
Definition: Polymeres quaternäres Ammoniumsalz, welches durch Umsetzung von Polyvinylalkohol mit 2,3-Epoxypropylamin erhältlich ist.

POLYQUATERNIUM-20
Definition: Polymeres quaternäres Ammoniumsalz, welches durch Umsetzung von Polyvinyloctadecylether mit 2,3-Epoxypropylamin erhältlich ist.

POLYQUATERNIUM-21 (CAS-Nummer: 102523-94-4)
Definition: Polysiloxan/Polydimethyldialkylammoniumacetat-Copolymer
Beispielsweise erhältlich als Abil® B 9905 (ex Goldschmidt-Degussa)

POLYQUATERNIUM-22 (CAS-Nummer: 53694-17-0)
Definition: Dimethyldiallylammoniumchlorid/Acrylsäure-Copolymer
Beispielsweise erhältlich als Merquat® 280 (ex Ondeo-Nalco)

POLYQUATERNIUM-24 (CAS-Nummer: 107987-23-5)
Definition: Polymeres quaternäres Ammoniumsalz aus der Umsetzung von Hydroxyethylcellulose mit einem mit Lauryldimethylammonium substituierten-Epoxid
Beispielsweise erhältlich als Quatrisoft

POLYQUATERNIUM-27
Definition: Blockcopolymer aus der Umsetzung von Polyquaternium-2 mit Polyquaternium-17.

POLYQUATERNIUM-28 (CAS-Nummer: 131954-48-8)
Definition: Vinylpyrrolidon/Methacrylamidopropyltrimethylammoniumchlorid-Copolymer
Beispielsweise erhältlich als Gafquat® HS-100 (ex GAF)

POLYQUATERNIUM-29
Definition: Chitosan, welches mit Propylenoxid umgesetzt und mit Epichlorhydrin quaternisiert wurde

POLYQUATERNIUM-30
Definition: Polymeres quaternäres Ammoniumsalz der Formel: $-[CH_2C(CH_3)(C(O)OCH_3)]_x-[CH_2C(CH_3)(C(O)OCH_2CH_2N^+(CH_3)_2CH_2COO^-)]_y-$

POLYQUATERNIUM-31 (CAS-Nummer. 136505-02-7)

POLYQUATERNIUM-32 (CAS-Nummer: 35429-19-7)
Definition: Polymer von N,N,N-Trimethyl-2-[(2-methyl-1-oxo-2-propenyl)oxy]-ethanaminiumchlorid mit 2-Propenamid

POLYQUATERNIUM-37 (CAS-Nummer: 26161-33-1)
Definition: Homopolymer von Methacryloyltrimethylchlorid
Beispielsweise erhältlich als Synthalen® CR (ex 3V Sigma)

POLYQUATERNIUM-44 (CAS-Nummer: 150595-70-5)
Definition: Quaternäres Ammoniumsalz des Copolymers von Vinylpyrrolidon und quaternisiertem Imidazolin
Beispielsweise erhältlich als Luviquat® Ultracare (ex BASF)

POLYQUATERNIUM-68 (CAS-Nummer: 827346-45-2)
Definition: Quaternisiertes Copolymer von Vinylpyrrolidon, Methacrylamid, Vinylimidazol und quatemisiertem Vinylimidazol
Beispielsweise erhältlich als Luviquai® Supreme (ex BASF)

Die kationischen Polymere weisen dabei nicht zwingend nur einen Textil-weichmachenden Effekt auf, sondern können zusätzlich einen weiteren textil- und/oder hautpflegenden Effekt aufweisen.

Unter einer hautpflegenden Verbindung wird eine Verbindung oder eine Mischung aus Verbindungen verstanden, die bei Kontakt eines Textils mit der festen, textil- und/oder hautpflegenden Zusammensetzung auf das Textil aufziehen und bei Kontakt des Textils mit Haut der Haut einen Vorteil verleihen verglichen mit einem Textil, welches nicht mit der erfindungemäßen textil- und/oder hautpflegenden Zusammensetzung behandelt wurde. Dieser Vorteil kann beispielsweise den Transfer der hautpflegenden Verbindung vom Textil auf die Haut, einen geringeren Wassertransfer von der Haut auf das Textil oder eine geringere Reibung auf der Hautoberfläche durch das Textil umfassen.

Eine hautpflegende Verbindung ist vorzugsweise hydrophob, kann flüssig oder fest sein und muss kompatibel mit den anderen Inhaltsstoffen der festen, textil- und oder hautpflegenden Zusammensetzung sein. Die hautpflegende Verbindung kann beispielsweise

a) Wachse wie Carnauba, Spermaceti, Bienenwachs, Lanolin, Derivate davon sowie Mischungen daraus;
b) hydrophobe Pflanzenextrakte, zum Beispiel pflanzliche Öle wie Avokadoöl, Olivenöl, Palmöl, Palmenkernöl, Rapsöl, Leinöl, Sojaöl, Erdnussöl, Korianderöl, Ricinusöl, Mohnöl, Kakaoöl, Kokosnüssöl, Kürbiskernöl, Weizenkeimöl, Sesamöl, Sonnenblumenöl, Mandelöl, Macadamianussöl, Aprikosenkernöl, Haselnussöl, Jojobaöl, Canolaöl sowie Mischungen daraus, Aloe oder Kamille;
c) höhere Fettsäuren wie Laurinsäure, Myristinsäure, Palmitinsäure, Stearinsäure, Behensäure, Ölsäure, Linolsäure, Linolensäure, Isostearinsäure oder mehrfach ungesättigte Fettsäuren;
d) höhere Fettalkohole wie Laurylalkohol, Cetylalkohol, Stearylalkohol, Oleylalkohol, Behenylalkohol oder 2-Hexadecanol,
e) Ester wie Cetyloctanoat, Lauryllactat, Myristyllactat, Cetyllactat, Isopropylmyristat, Myristylmyristat, Isopropylpalmitat, Isopropyladipat, Butylstearat, Decyloleat, Cholesterolisostearat, Glycerolmonostearat, Glyceroldistearat, Glyceroltristearat, Alkyllactat, Alkylcitrat oder Alkyltartrat;
f) Kohlenwasserstoffe wie Paraffine, Mineralöle, Squalan oder Squalen;

g) Lipide;
h) Vitamine wie Vitamin A, C und E oder Vitaminalkylester;
i) Phospholipide;
j) Sonnenschutzmittel wie Octylmethoxylcinnamat und Butylmethoxybenzoylmethan;
k) Silikonöle wie lineare oder cyclische Polydimethylsiloxane, Amino-, Alkyl-, Alkylaryl- oder Arylsubstituierte Silikonöle und
l) Mischungen daraus

umfassen.

Der wasserunlösliche Partikel enthält ein Parfüm.

Als Parfümöle bzw. Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind.

Die Menge an Parfüm in der textil- und/oder hautpflegenden Zusammensetzung beträgt dabei vorzugsweise zwischen 0,1 und 20 Gew.-%, insbesondere bevorzugt zwischen 1 und 10 Gew.-%, und ganz besonders bevorzugt zwischen 2 und 7 Gew.-%.

Weitere geeignete textilpflegende Verbindungen umfassen bevorzugt Fluoreszenzmittel, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Antistatika, Bügelhilfsmittel, UV-Absorber, Phobiermittel und/oder Imprägniermittel. Konkrete Beispiele für diese textilpflegenden Verbindungen finden sich bei der Beschreibung des erfindungsgemäßen Wasch- oder Reinigungsmittels und können auch in der festen, textil- und/oder hautpflegenden Zusammensetzung verwendet werden.

Die feste, textil- und/oder hautpflegende Zusammensetzung kann auch Mischungen der genannten Verbindungen enthalten.

Die Menge an textil- und/oder hautpflegender Verbindung in der textil- und/oder hautpflegenden Zusammensetzung beträgt 0,1 bis 20 Gew.-%.

Ein weiterer essentieller Bestandteil der festen, textil- und/oder hautpflegenden Zusammensetzung ist der wasserlösliche Partikel. Der wasserlösliche Partikel umfasst ein Kohlenhydrat, welches beispielsweise ausgewählt ist aus der Gruppe bestehend aus Dextrose, Fructose, Galactose, Isoglucose, Glucose, Saccharose, Raffinose und Mischungen daraus. Das eingesetzte Kohlenhydrat kann beispielsweise Kandiszucker, Teezucker, Kristallzucker oder Hagelzucker sein. Aufgrund seiner außergewöhnlichen Ästhetik und Akustik wird bevorzugt ein Zucker als wasserlöslicher Partikel bzw. als überwiegender Bestandteil eines wasserlöslichen Partikels eingesetzt.

Der wasserlösliche Partikel kann auch Mischungen aus einem Kohlenhydrat und anorganischen Alkalimetallsalzen wie beispielsweise Natriumchlorid, Kaliumchlorid, Natriumsulfat, Natriumcarbonat, Kaliumsulfat, Kaliumcarbonat, Natriumhydrogencarbonat, Kaliumhydrogencarbonat oder deren Mischungen, organischer Alkalimetallsalzen wie beispielsweise Natriumacetat, Kaliumacetat, Natriumcitrat, Natriumtartrat oder Kaliumnatriumtartrat, anorganischen Erdalkalimetallsalzen wie beispielsweise Calciumchlorid oder Magnesiumchlorid, organischen Erdalkalimetallsalzen wie beispielsweise wie Calciumlactat, organischen Säuren wie beispielsweise Citronensäure oder Weinsäure, Silikaten wie beispielsweise Wasserglas, Natriumsilikat oder Kaliumsilikat enthalten. Der wasserlösliche Partikel kann sich beispielsweise auch aus einem wasserlöslichen Trägerpartikel und weiteren Verbindungen zusammensetzen.

Es ist bevorzugt, dass der wasserlösliche Partikel und der wasserunlösliche Partikel jeweils Partikelgrößen im Bereich von 0,6 bis 30 mm, insbesondere 0,8 bis 20 mm und besonders bevorzugt 1 bis 10 mm, aufweist. Insbesondere bevorzugt umfasst der wasserlösliche Partikel Saccharose-Kristalle mit einer Partikelgröße von 1 bis 2 mm.

Es ist insbesondere bevorzugt, dass die Partikelgrößen der wasserlöslichen Partikel und der wasserunlöslichen Partikel in einem ähnlichen Bereich, dass heißt in der gleichen Größenordnung, liegen, um eine Entmischung der Partikel zu verhindern.

Dem Fachmann sind geeignete Methoden zur Bestimmung der Partikelgröße von Partikeln (zum Beispiel Pulver, Granulate oder Agglomerate) hinlänglich bekannt. Im Rahmen dieser Erfindung wurden die Partikelgrößen des wasserlöslichen Partikels, des wasserlöslichen Trägerpartikels, des wasserunlöslichen Partikels und/oder des wasserunlöslichen Trägerpartikels mittels Siebanalysen bestimmt.

Der wasserlösliche Partikel und der wasserunlösliche Partikel können jeweils optional weitere Inhaltsstoffe enthalten.

Um den ästhetischen Eindruck der textil- und/oder hautpflegenden Zusammensetzung zu verbessern, können die Partikel mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Wasch- oder Reinigungsmittel und gegen Licht sowie keine ausgeprägte Substantivität gegenüber Textilfasern, um diese nicht anzufärben.

Weiterhin können der wasserlösliche Partikel und/oder der wasserunlösliche Partikel einen Füllstoff, wie Silica, enthalten. Die Menge an Füllstoff in den Partikeln kann jeweils zwischen 0.1 und 10 Gew.-% betragen und beträgt bevorzugt 1 bis 5 Gew.-%..

Die Partikel und insbesondere der wasserunlösliche Partikel können jeweils zur Erhöhung des Glanzes auch ein Perlglanzmittel enthalten. Beispiele für geeignete Perlglanzmittel sind Ethylenglykolmono- und -distearat (zum Beispiel Cutina® AGS von Cognis) sowie PEG-3-distearat.

Zur Herstellung der textil- und/oder hautpflegenden Zusammensetzung werden zunächst die beiden Partikel in separaten Verfahren hergestellt bzw. bereitgestellt. Im Fall der wasserlöslichen Partikel kann das Verfahren der Bereitstellung lediglich im Erwerb der wasserlöslichen Verbindung mit der gewünschten Partikelgröße und Einfärbung der Verbindungen bestehen.

Zur Herstellung der wasserunlöslichen Partikel werden die wasserunlöslichen Trägerpartikel beispielsweise mittels Besprühen mit einer Flüssigkeit, die die textil- und/oder hautpflegende Verbindung enthält, behandelt/imprägniert.

Die textil- und/oder hautpflegende Zusammensetzung eignet sich insbesondere zum Konditionieren von textilen Flächengebilden und wird dazu zusammen mit einem herkömmlichen Wasch- oder Reinigungsmittel im (Haupt)Waschgang eines herkömmlichen Wasch- und Reinigungsprozesses mit den textilen Flächengebilden in Kontakt gebracht.

Die textil- und/oder hautpflegende Zusammensetzung kann in ein Wasch- oder Reinigungsmittel eingebracht werden.

Dazu wird ein festes Wasch- oder Reinigungsmittel mit 0,1 bis 20 Gew.-%, vorzugsweise 1 bis 10 Gew.-%, bezogen auf das gesamte, erfindungsgemäße Wasch- oder Reinigungsmittel, der erfindungsgemäßen textil- und/oder hautpflegenden Zusammensetzung gemischt.

Die erfindungsgemäßen Wasch- oder Reinigungsmittel enthalten neben der textil- und/oder hautpflegenden Zusammensetzung Tensid(e), wobei anionische, nichtionische, zwitterionische und/oder amphotere Tenside eingesetzt werden können. Bevorzugt sind aus anwendungstechnischer Sicht Mischungen aus anionischen und nichtionischen Tensiden. Der Gesamttensidgehalt eines Waschmittels liegt vorzugsweise unterhalb von 40 Gew.-% und besonders bevorzugt unterhalb von 35 Gew.-%, bezogen auf das gesamte Waschmittel.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise $C_{12-14}$-Alkohole mit 3 EO, 4 EO oder 7 EO, $C_{9-11}$-Alkohol mit 7 EO, $C_{13-15}$-Alkohole mit 3 EO, 5 EO, 7 EO oder 8 EO, $C_{12-18}$-Alkohole mit 3 EO, 5 EO oder 7 EO und Mischungen aus diesen, wie Mischungen aus $C_{12-14}$-Alkohol mit 3 EO und $C_{12-18}$-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Außerdem können als weitere nichtionische Tenside auch Alkylglykoside der allgemeinen Formel $RO(G)_x$ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4. Alkylglykoside sind bekannte, milde Tenside.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Auch nichtionische Tenside vom Typ der Aminoxide, beispielsweise N-Kokosalkyl-N,N-dimethyl-aminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel (VII),

$$\begin{array}{c} R^1 \\ | \\ R\text{-}CO\text{-}N\text{-}[Z] \end{array} \qquad (VII)$$

in der RCO für einen aliphatischen Acylrest mit 6 bis 22 Kohlenstoffatomen, R1 für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (VIII),

$$\begin{array}{c} R^1\text{-}O\text{-}R^2 \\ | \\ R\text{-}CO\text{-}N\text{-}[Z] \end{array} \qquad (VIII)$$

in der R für einen linearen oder verzweigten Alkyl- oder Alkenylrest mit 7 bis 12 Kohlenstoffatomen, $R^1$ für einen linearen, verzweigten oder cyclischen Alkylrest oder einen Arylrest mit 2 bis 8 Kohlenstoffatomen und $R^2$ für einen linearen, verzweigten oder cyclischen Alkylrest oder einen Arylrest oder einen Oxy-Alkylrest mit 1 bis 8 Kohlenstoffatomen steht, wobei $C_{1-4}$-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines Zuckers erhalten, beispielsweise Glucose, Fructose, Maltose, Lactose, Galactose, Mannose oder Xylose. Die N-Alkoxy- oder N-Aryloxy-substituierten Verbindungen können dann durch Umsetzung mit Fettsäuremethylestem in Gegenwart eines Alkoxids als Katalysator in die gewünschten Polyhydroxyfettsäureamide überführt werden.

Der Gehalt an nichtionischen Tensiden beträgt in den Wasch- oder Reinigungsmitteln bevorzugt 5 bis 30 Gew.-%, vorzugsweise 7 bis 20 Gew.-% und insbesondere 9 bis 15 Gew.-%, jeweils bezogen auf das gesamte Wasch- oder Reinigungsmittel .

Als anionische Tenside werden beispielsweise solche vom Typ der Sulfonate und Sulfate eingesetzt. Als Tenside vom Sulfonat-Typ kommen dabei vorzugsweise $C_{9-13}$-Alkylbenzolsulfonate, Olefinsulfonate, d.h. Gemische aus Alken- und Hydroxyalkansulfonaten sowie Disulfonaten, wie man sie beispielsweise aus $C_{12-18}$-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus $C_{12-18}$-Alkanen beispielsweise durch Sulfochlorierung oder Sulfoxidation mit anschließender Hydrolyse bzw. Neutralisation gewonnen werden. Ebenso sind auch die Ester von $\alpha$-Sulfofettsäuren (Estersulfonate), zum Beispiel die $\alpha$-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der $C_{12}$-$C_{18}$-Fettalkohole, beispielsweise aus Kokosfettalkohol, Talgfettalkohol, Lauryl-, Myristyl-, Cetyl- oder Stearylalkohol oder der $C_{10}$-$C_{20}$-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die $C_{12}$-$C_{16}$-Alkylsulfate und $C_{12}$-$C_{15}$-Alkylsulfate sowie $C_{14}$-$C_{15}$-Alkylsulfate bevorzugt. Auch 2,3-Alkylsulfate, welche als Handelsprodukte der

Shell Oil Company unter dem Namen DAN® erhalten werden können, sind geeignete Aniontenside.

Auch die Schwefelsäuremonoester der mit 1 bis 6 Mol Ethylenoxid ethoxylierten geradkettigen oder verzweigten $C_{7-21}$-Alkohole, wie 2-Methyl-verzweigte $C_{9-11}$-Alkohole mit im Durchschnitt 3,5 Mol Ethylenoxid (EO) oder $C_{12-18}$-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten $C_{8-18}$-Fettalkoholreste oder Mischungen aus diesen. Insbesondere bevorzugte Sulfosuccinate enthalten einen Fettalkoholrest; der sich von ethoxylierten Fettalkoholen ableitet, die für sich betrachtet nichtionische Tenside darstellen. Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl-oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Der Gehalt bevorzugter Wasch- oder Reinigungsmittel an anionischen Tensiden beträgt 2 bis 30 Gew.-%, vorzugsweise 4 bis 25 Gew.-% und insbesondere 5 bis 22 Gew.-%, jeweils bezogen auf das gesamte Wasch- oder Reinigungsmittel.

Zusätzlich zu der textil- und/oder hautpflegenden Zusammensetzung und den Tensiden können die Wasch- oder Reinigungsmittel weitere Inhaltsstoffe enthalten, die die anwendungstechnischen und/oder ästhetischen Eigenschaften des Wasch- oder Reinigungsmittels weiter verbessern. Im Rahmen der vorliegenden Erfindung enthalten bevorzugte Wasch- oder Reinigungsmittel zusätzlich einen oder mehrere Stoffe aus der Gruppe der Gerüststoffe, Bleichmittel, Bleichaktivatoren, Enzyme, Parfüme, Parfümträger, Fluoreszenzmittel, Farbstoffe, Schauminhibitoren, Silikonöle, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, neutrale Füllsalze sowie UV-Absorber.

Als Gerüststoffe, die in den Wasch- oder Reinigungsmitteln enthalten sein können, sind insbesondere Silikate, Aluminiumsilikate (insbesondere Zeolithe), Carbonate, Salze organischer Di- und Polycarbonsäuren sowie Mischungen dieser Stoffe zu nennen.

Geeignete kristalline, schichtförmige Natriumsilikate besitzen die allgemeine Formel $NaMSi_xO_{2x+1}$ $H_2O$, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl ß- als auch δ-Natriumdisilikate $Na_2Si_2O_5 \cdot yH_2O$ bevorzugt.

Einsetzbar sind auch amorphe Natriumsilikate mit einem Modul $Na_2O : SiO_2$ von 1 : 2 bis 1 : 3,3, vorzugsweise von 1 : 2 bis 1 : 2,8 und insbesondere von 1 : 2 bis 1 : 2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/ Verdichtung oder durch Übertrocknung hervorgerufen worden sein. Im Rahmen dieser Erfindung wird unter dem Begriff "amorph" auch "röntgenamorph" verstanden. Dies heißt, dass die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, dass die Produkte mikrokristalline Bereiche der Größe 10 bis einige Hundert nm aufweisen, wobei Werte bis maximal 50 nm und insbesondere bis maximal 20 nm bevorzugt sind. Insbesondere bevorzugt sind verdichtete/kompaktierte amorphe Silikate, compoundierte amorphe Silikate und übertrocknete röntgenamorphe Silikate.

Der eingesetzte feinkristalline, synthetische und gebundenes Wasser enthaltende Zeolith ist vorzugsweise Zeolith A und/oder P. Als Zeolith P wird Zeolith MAP® (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X); das von der Firma SASOL unter dem Markennamen VEGOBOND AX® vertrieben wird und

durch die Formel

$$nNa_2O \cdot (1\text{-}n)K_2O \cdot Al_2O_3 \cdot (2 - 2{,}5)SiO_2 \cdot (3{,}5 - 5{,}5)\ H_2O$$

$$n = 0{,}90 - 1{,}0$$

beschrieben werden kann. Der Zeolith kann als sprühgetrocknetes Pulver oder auch als ungetrocknete, von ihrer Herstellung noch feuchte, stabilisierte Suspension zum Einsatz kommen. Für den

Fall, dass der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten $C_{12}$-$C_{18}$-Fettalkoholen mit 2 bis 5 Ethylenoxidgruppen, $C_{12}$-$C_{14}$-Fettalkoholen mit 4 bis 5 Ethylenoxidgruppen oder ethoxylierten Isotridecanolen. Geeignete Zeolithe weisen eine mittlere Teilchengröße von weniger als 10 $\mu$m (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Organische Builder, welche in dem Wasch- oder Reinigungsmittel vorhanden sein können, umfassen Polycarboxylatpolymere wie Polyacrylate und Acrylsäure/Maleinsäure-Copolymere, Polyaspartate und monomere Polycarboxylate wie Citrate, Gluconate, Succinate oder Malonate, die bevorzugt als Natriumsalze eingesetzt werden.

Unter den als Bleichmittel dienenden, in Wasser $H_2O_2$ liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie $H_2O_2$ liefernde persaure Salze oder Persäuren, wie Perbenzoate, Peroxophthalate, Diperazelainsäure, Phthaloiminopersäure oder Diperdodecandisäure.

Um beim Waschen bei Temperaturen von 60 °C und darunter eine verbesserte Bleichwirkung zu erreichen, können Bleichaktivatoren in die Wasch- oder Reinigungsmittel eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zusätzlich zu den konventionellen Bleichaktivatoren oder an deren Stelle können auch sogenannte Bleichkatalysatoren in die Wasch- oder Reinigungsmittel eingearbeitet werden. Bei diesen Stoffen handelt es sich um bleichverstärkende Übergangsmetallsalze bzw. Übergangsmetallkomplexe wie beispielsweise Mn-, Fe-, Co-, Ru- oder Mo-Salenkomplexe oder -carbonylkomplexe. Auch Mn-, Fe-, Co-, Ru-, Mo-, Ti-, V- und Cu-Komplexe mit stickstoffhaltigen Tripod-Liganden sowie Co-, Fe-, Cu- und Ru-Amminkomplexe sind als Bleichkatalysatoren verwendbar.

Das Wasch- oder Reinigungsmittel kann Enzyme in verkapselter Form und/oder direkt in dem Wasch- oder Reinigungsmittel enthalten. Als Enzyme kommen insbesondere solche aus der Klassen der Hydrolasen wie der Proteasen, Esterasen, Lipasen bzw. lipolytisch wirkende Enzyme, Amylasen, Cellulasen bzw. andere Glykosylhydrolasen, Hemicellulase, Cutinasen, $\beta$-Glucanasen, Oxidasen, Peroxidasen, Mannanasen, Perhydrolasen und/oder Laccasen und Gemische der genannten Enzyme in Frage. Alle diese Hydrolasen tragen in der Wäsche zur Entfernung von Verfleckungen wie protein-, fett- oder stärkehaltigen Verfleckungen und Vergrauungen bei. Cellulasen und andere Glykosylhydrolasen können darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden. Besonders gut geeignet sind aus Bakterienstämmen oder Pilzen wie Bacillus subtilis, Bacillus licheniformis, Streptomyceus griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipase-haltige Mischungen bzw. Mischungen mit lipolytisch wirkenden Enzymen von besonderem Interesse. Beispiele für derartige lipolytisch wirkende

Enzyme sind die bekannten Cutinasen. Auch Peroxidasen oder Oxidasen haben sich in einigen Fällen als geeignet erwiesen. Zu den geeigneten Amylasen zählen insbesondere $\alpha$-Amylasen, Iso-Amylasen, Pullulanasen und Pektinasen. Als Cellulasen werden vorzugsweise Cellobiohydrolasen, Endoglucanasen und $\beta$-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffe adsorbiert sein, um sie gegen vorzeitige Zersetzung zu schützen. Der Anteil der Enzyme oder der Enzymgranulate direkt in dem Wasch- oder Reinigungsmittel kann beispielsweise etwa 0,01 bis 5 Gew.-%, vorzugsweise 0,12 bis etwa 2,5 Gew.-% betragen.

Es kann, beispielsweise bei speziellen Wasch- oder Reinigungsmitteln für Konsumenten mit Allergien und/ oder sensibler Haut, aber auch bevorzugt sein, dass das Wasch- oder Reinigungsmittel keine Enzyme enthält.

In einer Ausführungsform enthält das Wasch- oder Reinigungsmittel gegebenenfalls ein oder mehrere Parfüms in einer Menge von üblicherweise bis 10 Gew.-%, vorzugsweise 0,5 bis 7 Gew.-%, insbesondere 1 bis 3 Gew.-%. Dabei ist die Menge an eingesetztem Parfüm auch von der Art des Wasch- oder Reinigungsmittels abhängig. Es ist aber insbesondere bevorzugt, dass das Parfüm über die textil- und/oder hautpflegende Zusammensetzung in das Wasch- oder Reinigungsmittel eingebracht wird. Es ist allerdings auch möglich, dass das Wasch- oder Reinigungsmittel Parfüm enthält, welches nicht über die textil- und/oder hautpflegende Zusammensetzung in das Wasch- oder Reinigungsmittel eingebracht wird.

Um den ästhetischen Eindruck der Wasch- oder Reinigungsmittel zu verbessern, können sie (gegebenenfalls auch nur teilweise) mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Wasch- oder Reinigungsmittel und gegen Licht sowie keine ausgeprägte Substantivität gegenüber Textilfasern, um diese nicht anzufärben.

Als Schauminhibitoren, die in den Wasch- oder Reinigungsmitteln eingesetzt werden können, kommen beispielsweise Seifen, Paraffine oder Silikonöle in Betracht, die gegebenenfalls auf Trägermaterialien aufgebracht sein können.

Geeignete Soil-Release-Polymere, die auch als "Antiredepositionsmittel" bezeichnet werden, sind beispielsweise nichtionische Celluloseether wie Methylcellulose und Methylhydroxypropylcellulose mit einem Anteil an Methoxygruppen von 15 bis 30 Gew.-% und an Hydroxypropylgruppen von 1 bis 15 Gew.-%, jeweils bezogen auf den nichtionischen Celluloseether sowie die aus dem Stand der Technik bekannten Polymere der Phthalsäure und/oder Terephthalsäure bzw. von deren Derivaten, insbesondere Polymere aus Ethylenterephthalaten und/oder Polyethylen- und/oder Polypropylenglykolterephthalaten oder anionisch und/oder nichtionisch modifizierten Derivaten von diesen. Geeignete Derivate umfassen die sulfonierten Derivate der Phthalsäure- und Terephthalsäure-Polymere. Eine weitere Klasse an geeigneten Soil-Release-Polymeren, insbesondere für Baumwoll-haltige Textilien, stellen modifizierte, beispielsweise alkoxylierte und/oder quaternierte und/oder oxidierte, Polyamine dar. Die Polyamine sind beispielsweise Polyalkylenamine, wie Polyethylenamine, oder Polyalkylenimine, wie Polyethylenimine. Bevorzugte Beispiele für diese Klasse an Soil-Release-Polymeren sind ethoxylierte Polyethylenimine und ethoxylierte Polyethylenamine.

Optische Aufheller (so genannte "Weißtöner") können den Wasch- oder Reinigungsmitteln zugesetzt werden, um Vergrauungen und Vergilbungen der behandelten textilen Flächengebilde zu beseitigen. Diese Stoffe ziehen auf die Faser auf und bewirken eine Aufhellung und vorgetäuschte Bleichwirkung, indem sie unsichtbare Ultraviolettstrahlung in sichtbares längerwelliges Licht umwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiß ergibt. Geeignete Verbindungen stammen beispielsweise aus den Substanzklassen der 4,4'-Diamino-2,2'-stilbendisulfonsäuren (Flavonsäuren), 4,4'-Distyryl-biphenylen, Methylumbelliferone, Cumarine, Dihydrochinolinone, 1,3-Diarylpyrazoline, Naphthalsäureimide, Benzoxazol-, Benzisoxazol- und Benzimidazol-Systeme sowie der durch Heterocyclen substituierten Pyrenderivate. Die optischen Aufheller werden üblicherweise in Mengen zwischen 0% und 0,3 Gew.-%, bezogen auf das fertige Wasch- oder Reinigungsmittel, eingesetzt.

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, zum Beispiel abgebaute Stärke, Aldehydstärken usw. Auch Polyvinylpyrrolidon ist brauchbar. Bevorzugt werden jedoch Celluloseether wie Carboxymethylcellulose (Na-Salz), Methylcellulose, Hydroxyalkylcellulose und Mischether wie Methylhydroxyethylcellulose, Methylhydroxypropylcellulose, Methylcarboxymethylcellulose und deren Gemische in Mengen von 0,1 bis 5 Gew.-%, bezogen auf die Wasch- oder Reinigungsmittel, eingesetzt.

Um während des Waschens und/oder des Reinigens von gefärbten Textilien die Farbstoffablösung und/oder die Farbstoffübertragung auf andere Textilien wirksam zu unterdrücken, kann das Wasch- oder Reinigungsmittel einen

Farbübertragungsinhibitor enthalten. Es ist bevorzugt, dass der Farbübertragungsinhibitor ein Polymer oder Copolymer von cyclischen Aminen wie beispielsweise Vinylpyrrolidon und/oder Vinylimidazol ist. Als Farbübertragungsinhibitor geeignete Polymere umfassen Polyvinylpyrrolidon (PVP), Polyvinylimidazol (PVI), Copolymere von Vinylpyrrolidon und Vinylimidazol (PVP/PVI), Polyvinylpyridin-N-oxid, Poly-N-carboxymethyl-4-vinylpyridiumchlorid sowie Mischungen daraus. Besonders bevorzugt werden Polyvinylpyrrolidon (PVP), Polyvinylimidazol (PVI) oder Copolymere von Vinylpyrrolidon und Vinylimidazol (PVP/PVI) als Farbübertragungsinhibitor eingesetzt. Die eingesetzten Polyvinylpyrrolidone (PVP) besitzen bevorzugt ein mittleres Molekular gewicht von 2.500 bis 400.000 und sind kommerziell von ISP Chemicals als PVP K 15, PVP K 30, PVP K 60 oder PVP K 90 oder von der BASF als Sokalan® HP 50 oder Sokalan® HP 53 erhältlich. Die eingesetzten Copolymere von Vinylpyrrolidon und Vinylimidazol (PVP/PVI) weisen vorzugsweise ein Molekulargewicht im Bereich von 5.000 bis 100.000 auf. Kommerziell erhältlich ist ein PVP/PVI-Copolymer beispielsweise von der BASF unter der Bezeichnung Sokalan® HP 56.

Die Menge an Farbübertragungsinhibitor bezogen auf die Gesamtmenge des Wasch- oder Reinigungsmittel liegt bevorzugt von 0,01 bis 2 Gew.-%, vorzugsweise von 0,05 bis 1 Gew.-% und mehr bevorzugt von 0,1 bis 0,5 Gew.-%.

Alternativ können aber auch enzymatische Systeme, umfassend eine Peroxidase und Wasserstoffperoxid beziehungsweise eine in Wasser Wasserstoffperoxid-liefernde Substanz, als Farbübertragungsinhibitor eingesetzt werden. Der Zusatz einer Mediatorverbindung für die Peroxidase, zum Beispiel eines Acetosyringons, eines Phenolderivats oder eines Phenotiazins oder Phenoxazins, ist in diesem Fall bevorzugt, wobei auch zusätzlich die oben genannten polymeren Farbübertragungsinhibitoren eingesetzt werden können.

Da textile Flächengebilde, insbesondere aus Reyon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können die Wasch- oder Reinigungsmittel synthetische Knitterschutzmittel enthalten. Hierzu zählen beispielsweise synthetische Produkte auf der Basis von Fettsäuren, Fettsäureestern, Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Zur Bekämpfung von Mikroorganismen können die Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat, wobei bei den erfindungemäßen Wasch- oder Reinigungsmitteln auch gänzlich auf diese Verbindungen verzichtet werden kann.

Die erfindungsgemäßen Wasch- oder Reinigungsmittel können Konservierungsmittel enthalten, wobei vorzugsweise nur solche eingesetzt werden, die kein oder nur ein geringes hautsensibilisierendes Potential besitzen. Beispiele sind Sorbinsäure und seine Salze, Benzoesäure und seine Salze, Salicylsäure und seine Salze, Phenoxyethanol, 3-Iod-2-propynylbutylcarbamat, Natrium N-(hydroxymethyl)glycinat, Biphenyl-2-ol sowie Mischungen davon.

Um unerwünscht, durch Sauerstoffeinwirkung und andere oxidative Prozesse verursachte Veränderungen an den Wasch- oder Reinigungsmittel und/oder den behandelten textilen Flächengebilden zu verhindern, können die Wasch- oder Reinigungsmittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite, Phosphonate und Vitamin E.

Ein erhöhter Tragekomfort kann aus der zusätzlichen Verwendung von Antistatika resultieren, die den Wasch- oder Reinigungsmitteln beigefügt werden. Antistatika vergrößern die Oberflächenleitfähigkeit und ermöglichen damit ein verbessertes Abfließen gebildeter Ladungen. Äußere Antistatika sind in der Regel Substanzen mit wenigstens einem hydrophilen Molekülliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsäureester) und schwefelhaltige (Alkylsulfonate, Alkylsulfate) Antistatika unterteilen. Lauryl- (bzw. Stearyl-)dimethylbenzylammoniumchloride eignen sich als Antistatika für textile Flächengebilde bzw. als Zusatz zu Wasch- oder Reinigungsmitteln, wobei zusätzlich ein Avivageeffekt erzielt wird.

Zur Verbesserung des der Wiederbenetzbarkeit der behandelten textilen Flächengebilde und zur Erleichterung des Bügelns der behandelten textilen Flächengebilde können in den Wasch- oder Reinigungsmitteln beispielsweise Silikonderivate eingesetzt werden. Diese verbessern zusätzlich das Ausspülverhalten der Wasch- oder Reinigungsmittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H- und/oder Si-Cl-Bindungen aufweisen. Die Viskositäten der bevorzugten Silikone liegen bei 25°C im Bereich zwischen 100 und 100.000 mPas, wobei die Silikone in Mengen zwischen 0,2 und 5 Gew.-%, bezogen auf das gesamte Wasch- oder Reinigungsmittel eingesetzt werden können.

Schließlich können die Wasch- oder Reinigungsmittel auch UV-Absorber enthalten, die auf die behandelten textilen Flächengebilde aufziehen und die Lichtbeständigkeit der Fasern verbessern. Verbindungen, die diese gewünschten Eigenschaften aufweisen, sind beispielsweise die durch strahlungslose Desaktivierung wirksamen Verbindungen

und Derivate des Benzophenons mit Substituenten in 2- und/oder 4-Stellung. Weiterhin sind auch substituierte Benzotriazole, in 3-Stellung Phenyl-substituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

Um die durch Schwermetalle katalysierte Zersetzung bestimmter Waschmittel-Inhaltsstoffe zu vermeiden, können Stoffe eingesetzt werden, die Schwermetalle komplexieren. Geeignete Schwermetallkomplexbildner sind beispielsweise die Alkalisalze der Ethylendiamintetraessigsäure (EDTA) oder der Nitrilotriessigsäure (NTA) sowie Alkalimetallsalze von anionischen Polyelektrolyten wie Polymaleaten und Polysulfonaten.

Eine bevorzugte Klasse von Komplexbildnern sind die Phosphonate, die in bevorzugten Wasch- oder Reinigungsmittel in Mengen von 0,01 bis 2,5 Gew.-%, vorzugsweise 0,02 bis 2 Gew.-% und insbesondere von 0,03 bis 1,5 Gew.-% enthalten sind. Zu diesen bevorzugten Verbindungen zählen insbesondere Organophosphonate wie beispielsweise 1-Hydroxyethan-1,1-diphosphonsäure (HEDP), Aminotri(methylenphosphonsäure) (ATMP), Diethylentriaminpenta(methylenphosphonsäure) (DTPMP bzw. DETPMP) sowie 2-Phosphonobutan-1,2,4-tricarbonsäure (PBS-AM), die zumeist in Form ihrer Ammonium- oder Alkalimetallsalze eingesetzt werden.

Zusätzlich können noch neutrale Füllsalze wie Natriumsulfat oder Natriumcarbonat in den festen Wasch- oder Reinigungsmitteln enthalten sein.

Die erfindungsgemäßen Wasch- oder Reinigungsmittel können insbesondere zum Reinigen und Konditionieren von textilen Flächengebilden verwendet werden.

Zur Herstellung der erfindungsgemäßen Wasch- oder Reinigungsmittel wird zunächst das Wasch- oder Reinigungsmittel ohne die textil- und/oder hautpflegende Zusammensetzung nach bekannten Verfahren; welche beispielsweise Trocknungsschritte, Mischungsschritte, Verdichtungsschritte, Formgebungsschritte und/oder die nachträgliche Zugabe wärmeempfindlicher Inhaltsstoffe ("Post Addition") umfassen können, hergestellt. Anschließend wird das erhaltene Produkt mit einer festen, textil- und/oder hautpflegende Zusammensetzung vermischt. Zur Herstellung von Wasch- oder Reinigungsmittelformkörpern können sich dem Mischungsschritt weitere Verdichtungs- und/oder Formgebungsschritte anschließen.

**Beispiele**

Beispiel 1

Wasserlöslicher Partikel

Zur Herstellung einer erfindungsgemäßen textil- und/oder hautpflegenden Zusammensetzung E1 wurde Industriezucker mit einer Partikelgröße von 1 bis 3 mm mit dem Farbstoff Pigment Red 5 rot eingefärbt.

Wasserunlöslicher Partikel

Parallel wurden granulierte Betonit-Partikel (Laundrosil® DGA ex Südchemie) mit einer Partikelgröße von 1 bis 3 mm mit demselben Farbstoff (Pigment Red 5) wie der Industriezucker rot eingefärbt und anschließend mit einem Parfüm besprüht.

Die beiden Partikel wurden anschließend in einem solchen Verhältnis gemischt, dass die fertige, erfindungsgemäße textil- und/oder hautpflegende Zusammensetzung 52 Gew.-% Industriezucker, 45 Gew.-% Bentonit, 2,9 Gew.-% Parfüm und 0,1 Gew.-% Farbstoff enthielt.

Zum Vergleich der Duftintensität eines herkömmlichen flüssigen Weichspülers (Gehalt an Textilweichmachendem Diesterquat: 15 Gew.-%) mit den festen, textil- und/oder hautpflegenden Zusammensetzungen wurde Frotteegewebe einerseits mit einem festen, im Handel erhältlichen Waschmittel und dem herkömmlichen Weichspüler sowie andererseits mit dem selben Waschmittel und der festen textil- und/oder hautpflegenden Zusammensetzungen E1 in einer Waschmaschine (Miele Novotronic W 985) behandelt. Nach hängender Trocknung wurde die Duftintensität bestimmt:

| Zusammensetzung | Feuchte, frisch gewaschene Wäsche | Nach 1 Tag an trockener Wäsche | Nach 7 Tagen an trockener Wäsche |
|---|---|---|---|
| Vergleich | 1,2 | 0,9 | 0,7 |

(fortgesetzt)

| Zusammensetzung | Feuchte, frisch gewaschene Wäsche | Nach 1 Tag an trockener Wäsche | Nach 7 Tagen an trockener Wäsche |
|---|---|---|---|
| **E1** | 2,3 | 1,1 | 1,1 |
| Bewertung: 0 = schwach bis 4 = stark<br>Anzahl der bewertenden Personen: 7 | | | |

Weiterhin zeigten die erfindungsgemäßen textil- und/oder hautpflegenden Zusammensetzungen im Vergleich mit Wasser einen Textil-weichmachenden Effekt. Die mit Wasser bzw. mit den textil- und/oder hautpflegenden Zusammensetzungen behandelten Gewebe wurden dazu nach der Behandlung und hängender Trocknung von einem Panel bestehend aus 5 Personen abgegriffen. Während sich für mit Wasser behandelte Gewebe ein Weichheitswert von 1,9 (0 = hart bis 6 = weich) ergab, ergaben mit **E1** behandelte Gewebe einen Weichheitswert von 3,0.

Zusätzlich sind die erfindungsgemäßen textil- und/oder hautpflegenden Zusammensetzungen in der Lage, die Härte von Wasser zu reduzieren. Diese Bestimmung erfolgte mit Analysestäbchen "Gesamthärtetest" (Fa. Merck) gemäß den Vorschriften des Herstellers.

Bessere Ergebnisse für den Weichheitswert wurden für erfindungsgemäße textil- und/oder hautpflegende Zusammensetzungen erhalten, bei denen die wasserunlöslichen Bentonit-Partikel zusätzlich mit einer Textil-weichmachenden Verbindungen (**E2**: 4 Gew.-% Polydimethylsiloxan, **E3**: 5 Gew.-% Polyquaternium-7 und **E4**: 5 Gew.-% Polyquaternium-10) besprüht bzw. behandelt wurden. Die Gew.-% geben die Gewichtsmenge der Textil-weichmachenden Verbindungen in der fertigen textil- und/oder hautpflegenden Zusammensetzung an. Die Mengen an Industriezucker bzw. Bentonit in der fertigen textil- und/oder hautpflegenden Zusammensetzung wurden entsprechend jeweils um 2 Gew.-% bzw. 2,5 Gew.-% reduziert.

Eine weitere textil- und/oder hautpflegende Zusammensetzung wurde analog wie oben beschrieben erhalten, indem die wasserunlöslichen Bentonit-Partikel zusätzlich mit einer Lösung, die den optischen Aufheller Tinopal® CBS-X (ex Ciba) enthielt, besprüht wurden und anschließend diese wasserunlöslichen Partikel mit den wasserlöslichen Partikeln gemischt wurden. Die fertige, erfindungsgemäße textil- und/oder hautpflegende Zusammensetzung **E5** enthielt 52 Gew.-% Industriezucker, 44,95 Gew.-% Bentonit, 2,9 Gew.-% Parfüm, 0,05 Gew.-% optischen Aufheller und 0,1 Gew.-% Farbstoff. Textile Flächengebilde, die mit der textil- und/oder hautpflegende Zusammensetzung **E5** behandelt wurden, wiesen einen erhöhten Weißeindruck auf.

Zur Herstellung eines erfindungsgemäßen Wasch- oder Reinigungsmittels wurde ein festes, unparfümiertes Wasch- oder Reinigungsmittel mit 10 Gew.-% (bezogen auf die Gesamtmenge an fertigem Wasch- oder Reinigungsmittel) der textil- und/oder hautpflegenden Zusammensetzung **E1** gemischt.

Das erfindungsgemäßen Wasch- oder Reinigungsmittel zeigte gute reinigende und konditionierende Eigenschaften.

Weder bei separater Anwendung der textil- und/oder hautpflegenden Zusammensetzung noch eingebracht in einem Wasch- oder Reinigungsmittel wurden Kalkablagerungen auf der Wäsche und/oder Ablagerungen/Rückstände in der Einspülkammer der Waschmaschinen beobachtet.

## Patentansprüche

1. Feste, textil- und/oder hautpflegende Zusammensetzung, umfassend einen wasserlöslichen Partikel und einen wasserunlöslichen Partikel, der einen wasserunlöslichen Träger und 0,1 bis 20 Gew.-% einer textil- und/oder haufpflegenden Verbindung enthält, wobei der wasserlösliche Partikel ein Kohlenhydrat umfasst, die textil- und/oder hautpflegende Verbindung ein Parfum enthält und der wasserunlösliche Träger ein Textil-weichmachender Ton ist.

2. Feste, textil- und/oder hautpflegende Zusammensetzung gemäß Anspruch 1, **dadurch gekennzeichnet, dass** das Kohlenhydrat ausgewählt ist aus der Gruppe bestehend aus Dextrose, Fructose, Galactose, Isoglucose, Glucose, Saccharose, Raffinose und Mischungen daraus.

3. Feste, textil- und/oder hautpflegende Zusammensetzung gemäß einem der Ansprüche 1 oder 2, **dadurch gekennzeichnet, dass** die feste, textil- und/oder hautpflegende Zusammensetzung 10 bis 90 Gew.-%, bevorzugt 40 bis 60 Gew.-% und ganz besonders bevorzugt 45 bis 55 Ges.-%, an wasserlöslichen Partikeln enthält.

4. Feste, textil- und/oder hautpflegende Zusammensetzung gemäß Anspruch 1, **dadurch gekennzeichnet, dass** der der Textil-weichmachende Ton ein Bentonit ist.

5. Feste, textil- und/oder hautpflegende Zusammensetzung gemäß einem der Ansprüche 1 bis 4, **dadurch gekennzeichnet, dass** die Zusammensetzung eine weitere textil- und/oder haulpflegende Verbindung enthält, die ausgewählt ist aus der Gruppe bestehend aus Textil-weichmachenden Verbindungen, Fluoreszenzmitteln, Antiredepositionsmilteln, optischen Aufhellern, Vergrauungsinhibitoren, Einlaufverhinderern, Knitterschutzmitteln, Farbübertragungsinhibitoren, antimikrobiellen Wirkstoffen, Germiziden, Fungiziden, Antioxidantien, Antistatika, Bügelhilfsmitteln, UV-Absorbern, Phobiermitteln. Imprägniermitteln, Haut-pflegenden Verbindungen und Mischungen daraus.

6. Feste, textil- und/oder hautpflegende zusammensetzung gemäß Anspruch 1, **dadurch gekennzeichnet, dass** die Menge an Parfüm 0,1 bis 20 Gew.-%, bevorzugt 1 bis 10 Gew.-% und insbesondere bevorzugt 2 bis 7 Gew.-% beträgt.

7. Feste, textil- und/oder hautpflegende Zusammensetzung gemäß Anspruch 5, **dadurch gekennzeichnet, dass** der wasserunlösliche Partikel als Textil-weichmachende Verbindung ein Textil-weichmachendes Polymer ausgewählt aus der Gruppe bestehend aus Polysiloxanen, kationischen Polymeren und Mischungen daraus enthält.

8. Feste, textil- und/oder hautpflegende Zusammensetzung gemäß einem der Ansprüche 1 bis 7, **dadurch gekennzeichnet, dass** der wasserunlösliche Partikel zusätzlich Inhaltsstoffe ausgewählt aus der Gruppe bestehend aus Farbstoffen, Füllstoffen, Perlglanzmitteln und Mischungen daraus enthält.

9. Feste, textil- und/oder hautpflegende Zusammensetzung gemäß einem der Ansprüche 1 bis 8, **dadurch gekennzeichnet, dass** der wasserlösliche Partikel und/oder der wasserunlösliche Partikel jeweils eine Partikelgröße im Bereich von 0,6 bis 30 mm, insbesondere 0,8 bis 20 mm und besonders bevorzugt 1 bis 10 mm, aufweisen.

10. Verfahren zur Herstellung einer festen, textil- und/oder hautpflegenden Zusammensetzung, umfassend einen wasserlöslichen Partikel und einen wasserunlöslichen Partikel, der einen wasserunlöslichen Träger und 0,1 bis 20 Gew.-% einer textil- und/oder hautpflegenden Verbindung enthält, wobei der wasserlösliche Partikel ein Kohlenhydrat umfasst, die textil- und/oder hautpflegende Verbindung ein Parfüm enthält und der wasserunlösliche Träger ein Textil-weichmachender Ton ist, bei dem der wasserlösliche Partikel und der wasserunlösliche Partikel gemischt werden.

11. Wasch- oder Reinigungsmittel, umfassend eine feste, textil- und/oder hautpflegenden Zusammensetzung gemäß einem der Ansprüche 1 bis 9.

12. Verfahren zur Herstellung eines Wasch- oder Reinigungsmittel gemäß Anspruch 11, bei dem ein festes Wasch- oder Reinigungsmittel mit einer festen, textil- und/oder hautpflegenden Zusammensetzung gemäß einem der Ansprüche 1 bis 9 gemisch wird.

## Claims

1. A solid, textile care and/or skin care composition, comprising a water-soluble particle and a water-insoluble particle, which contains a water-insoluble carrier and 0.1 to 20% by weight of a textile care and/or skin care compound, wherein the water-soluble particle comprises a carbohydrate, the textile care and/or skin care compound contains a perfume and the water-insoluble carrier is a textile softener clay.

2. The solid, textile care and/or skin care composition according to claim 1, **characterized in that** the carbohydrate is selected from the group consisting of dextrose, fructose, galactose, isoglucose, glucose, saccharose, raffinose and mixtures thereof.

3. The solid, textile care and/or skin care composition according to any of claims 1 or 2, **characterized in that** the solid, textile care and/or skin care composition contains 10 to 90% by weight, preferably 40 to 60% by weight and most preferably 45 to 55% by weight of water-soluble particles.

4. The solid, textile care and/or skin care composition according to claim 1, **characterized in that** the textile softener clay is a bentonite.

5. The solid, textile care and/or skin care composition according to any of claims 1 to 4, **characterized in that** the composition contains a further textile care and/or skin care compound, which is selected from the group consisting of textile softening compounds, fluorescence agents, anti-redeposition agents, optical brighteners, greying inhibitors,

shrinkage preventing agents, anti-crease agents, color transfer inhibitors, anti-microbial active substances, germicides, fungicides, anti-oxidants, antistatic agents, ironing aids, UV absorbers, proofing agents, impregnation agents, skin care compounds and mixtures thereof.

**6.** The solid, textile care and/or skin care composition according to claim 1, **characterized in that** the amount of perfume is from 0.1 to 20% by weight, preferably 1 to 10% by weight and more preferably 2 to 7% by weight.

**7.** The solid, textile care and/or skin care composition according to claim 5, **characterized in that** the water-insoluble particle contains a textile softening polymer selected from the group consisting of polysiloxanes, cationic polymers and mixtures thereof as a textile softening compound.

**8.** The solid, textile care and/or skin care composition according to any of claims 1 to 7, **characterized in that** the water-insoluble particle further contains ingredients selected from the group consisting of coloring agents, fillers, pearlescent agents and mixtures thereof.

**9.** The solid, textile care and/or skin care composition according to any of claims 1 to 8, **characterized in that** the water-soluble particle and/or the water-insoluble particle each have a particle size in the range from 0.6 to 30 mm, in particular 0.8 to 20 mm and more preferably 1 to 10 mm.

**10.** A method for preparing a solid, textile care and/or skin care composition, comprising a water-soluble particle and a water-insoluble particle, which contains a water-insoluble carrier and 0.1 to 20% by weight of a textile care and/or skin care compound, wherein the water-soluble particle comprises a carbohydrate, the textile care and/or skin care compound contains a perfume and the water-insoluble carrier is a textile softening clay, in which the water-soluble particle and the water-insoluble particle are mixed.

**11.** A washing or cleaning agent, comprising a solid, textile care and/or skin care composition according to any of claims 1 to 9.

**12.** A method for preparing a washing or cleaning agent according to claim 11, in which a solid washing or cleaning agent is mixed with a solid, textile care and/or skin care composition according to any of claims 1 to 9.

## Revendications

**1.** Composition solide pour le soin des textiles et/ou de la peau, comprenant une particule soluble dans l'eau et une particule insoluble dans l'eau qui contient un support insoluble dans l'eau et un composé pour le soin des textiles et/ou de la peau à concurrence de 0,1 à 20 % en poids, la particule soluble dans l'eau comprenant un hydrate de carbone, le composé pour le soin des textiles et/ou de la peau contenant un parfum et le support insoluble dans l'eau étant une argile assouplissant les textiles.

**2.** Composition solide pour le soin des textiles et/ou de la peau selon la revendication 1, **caractérisée en ce que** l'hydrate de carbone est choisi parmi le groupe constitué par le dextrose, le fructose, le galactose, l'isoglucose, le glucose, le saccharose, le raffinose et leurs mélanges.

**3.** Composition solide pour le soin des textiles et/ou de la peau selon la revendication 1 ou 2, **caractérisée en ce que** la composition solide pour le soin des textiles et/ou de la peau contient des particules solubles dans l'eau à concurrence de 10 à 90 % en poids, de préférence à concurrence de 40 à 60 % en poids et de manière tout particulièrement préférée à concurrence de 45 à 55 % en poids.

**4.** Composition solide pour le soin des textiles et/ou de la peau selon la revendication 1, **caractérisée en ce que** l'argile assouplissant les textiles est une bentonite.

**5.** Composition solide pour le soin des textiles et/ou de la peau selon l'une quelconque des revendications 1 à 4, **caractérisée en ce que** la composition contient un composé supplémentaire pour le soin des textiles et/ou de la peau, qui est choisi parmi le groupe constitué par des composés assouplissant les textiles, des agents fluorescents, des agents anti-redéposition, des azurants optiques, des inhibiteurs de frisaillement, des agents empêchant le rétrécissement, des agents anti- froissements, des inhibiteurs du transfert des couleurs, des substances actives antimicrobiennes, des germicides, des fongicides, des antioxydants, des agents antistatiques, des agents facilitant

le repassage, des agents absorbant l'ultraviolet, des agents rendant hydrophobe, des agents d'imprégnation, des composés pour les soins de la peau, ainsi que leurs mélanges.

6. Composition solide pour le soin des textiles et/ou de la peau selon la revendication 1, **caractérisée en ce que** la quantité de parfum s'élève de 0,1 à 20 % en poids, de préférence de 1 à 10 % en poids et de manière particulièrement préférée de 2 à 7 % en poids.

7. Composition solide pour le soin des textiles et/ou de la peau selon la revendication 5, **caractérisée en ce que** la particule insoluble dans l'eau contient, à titre de composé assouplissant les textiles, un polymère assouplissant les textiles choisi parmi le groupe constitué par des polysiloxanes, des polymères cationiques, ainsi que leurs mélanges.

8. Composition solide pour le soin des textiles et/ou de la peau selon l'une quelconque des revendications 1 à 7, **caractérisée en ce que** la particule insoluble dans l'eau contient en outre des constituants choisis parmi le groupe constitué par des colorants, des matières de charge, des agents conférant un reflet nacré, ainsi que leurs mélanges.

9. Composition solide pour le soin des textiles et/ou de la peau selon l'une quelconque des revendications 1 à 8, **caractérisée en ce que** la particule soluble dans l'eau et/ou la particule insoluble dans l'eau présentent respectivement une granulométrie dans la plage de 0,6 à 30 mm, en particulier de 0,8 à 20 mm, et de manière particulièrement préférée de 1 à 10 mm.

10. Procédé pour la préparation d'une composition solide pour le soin des textiles et/ou de la peau comprenant une particule soluble dans l'eau et une particule insoluble dans l'eau qui contient un support insoluble dans l'eau et un composé pour le soin des textiles et/ou de la peau à concurrence de 0,1 à 20 % en poids, la particule soluble dans l'eau comprenant un hydrate de carbone, le composé pour le soin des textiles et/ou de la peau contenant un parfum et le support insoluble dans l'eau étant une argile assouplissant les textiles, procédé dans lequel on mélange la particule soluble dans l'eau et la particule insoluble dans l'eau.

11. Agent de lavage ou de nettoyage comprenant une composition solide pour le soin des textiles et/ou de la peau selon l'une quelconque des revendications 1 à 9.

12. Procédé pour la préparation d'une composition solide pour le soin des textiles et/ou de la peau selon la revendication 11, dans lequel on mélange un agent de lavage ou de nettoyage solide avec une composition solide pour le soin des textiles et/ou de la peau selon l'une quelconque des revendications 1 à 9.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- CTFA International Cosmetic Ingredient Dictionary. Cosmetic, Toiletry, and Fragrance Association, 1991